# EUROPEAN PATENT SPECIFICATION

(11) **EP 4 009 973 B1**
(45) Date of publication and mention of the grant of the patent: **29.01.2025**
(21) Application number: 20849662.0
(22) Date of filing: 07.08.2020
(51) Int. Cl.: A61K 9/08, A61K 31/473, A61K 31/485, C07D 215/14, A61K 47/02, A61K 47/10, A61K 47/40, C07D 221/18

(54) **PHARMACEUTICAL COMPOSITIONS OF (6AS)-6-METHYL-5,6,6A,7-TETRAHYDRO-4H-DIBENZO[DE,G]QUINOLINE-10,11-DIOL**
PHARMAZEUTISCHE ZUSAMMENSETZUNGEN VON (6AS) -6-METHYL-5,6,6A,7-TETRAHYDRO-4H-DIBENZO[DE,G]CHINOLIN-10,11-DIOL
COMPOSITIONS PHARMACEUTIQUES DE (6AS)-6-MÉTHYL -5,6,6A,7-TÉTRAHYDRO-4 H-DIBENZO[DE,G]QUINOLÉINE-10,11-DIOL

(30) Priority: 07.08.2019 US 201962883910 P
(43) Date of publication of application: 15.06.2022
(73) Proprietor: Aclipse One, Inc., Radnor, PA 19087 (US)
(72) Inventor: SHAN, Ning, Chandler, Arizona 85249 (US)
(74) Representative: Herrero & Asociados, S.L.
(86) International application number: PCT/US2020/045338
(87) International publication number: WO 2021/026421

(56) References cited:
- WO-A1-2019/222103
- US-A1- 2009 047 234
- US-A1- 2018 098 981
- SAARI ET AL: "Synthesis and biological activity of (6aS)-10,11-dihydroxyaporphine, the optical antipode of apomorphine", NOTES JOURNAL OF MEDICINAL CHEMISTRY, vol. 16, no. 2, 1 January 1973 (1973-01-01), pages 171 - 172, XP055655724, DOI: 10.1021/jm00260a022
- BREWSTER ET AL: "Cyclodextrins as pharmaceutical solubilizers", ADVANCED DRUG DELIVERY REVIEWS, ELSEVIER, AMSTERDAM , NL, vol. 59, no. 7, 24 August 2007 (2007-08-24), pages 645 - 666, XP022211985, ISSN: 0169-409X, DOI: 10.1016/J.ADDR.2007.05.012

## Description

PHARMACEUTICAL COMPOSITIONS OF (6aS)-6-METHYL-5,6,6a,7-TETRAHYDRO-4H-DIBENZO[de,g]QUINOLINE-10,11-DIOL

### CROSS-REFERENCE TO RELATED APPLICATION

This application claims the benefit of U.S. Provisional Application Number 62/883,910, filed August 7, 2019.

### Field of Invention

This invention relates to pharmaceutical compositions for enhancing the solubility of (6aS)-6-methyl-5,6,6a,7-tetrahydro-4H-dibenzo[de,g]quinoline-10,11-diol and salt forms thereof.

### Background

Active pharmaceutical ingredients (APls) in pharmaceutical compositions can be delivered for systemic exposure via a variety of different route of administrations. Routes of administration are generally classified by the location at which the substance is applied. One common route of administration is via parenteral route. This includes intravenous administration, intramuscular administration and subcutaneous administration. For parenteral administrations, APIs are usually prepared in liquid solutions and delivered using a syringe or an automatic injectable device. Another common route of administration is via oral route. For oral administrations, APIs are typically formulated in the stable pharmaceutical dosage and orally delivered to the patients. Those oral dosage forms can be solid dosage forms, semi-solids, or liquid dosage forms.

To achieve desired concentration of drugs in systemic circulation for desired pharmacological response, solubility is one of the most important physicochemical properties in pharmaceutical development and drug delivery.

For parenteral administrations, the desired solubility profile of an API can facilitate the development of an injectable dosage form, simplify the manufacturing process, ensure uniformity of dosage forms, and reduce the risk of adverse effect at the injection site.

For oral administrations, a liquid dosage form with desired solubility can significantly improve the oral bioavailability of the API. For those patients with dysphagia or swallowing difficulties, a liquid dosage form can also improve patience compliance.

Low aqueous solubility is the major problem encountered with formulation development of new chemical entities as well as for the generic development. More than 40% of new chemical entities (NCEs) developed in pharmaceutical industry are practically insoluble in water. Solubility is a major challenge for pharmaceutical scientists. Saari et al., Journal of Medicinal Chemistry (1973) Vol. 16, No. 2, pages 171-172, discloses the synthesis and biological activity of (6aS)-6-methyl-5,6,6a,7-tetrahydro-4H-dibenzo[de,g]quinoline-10,11-diol.

(6aS)-6-methyl-5,6,6a,7-tetrahydro-4H-dibenzo[de,g]quinoline-10,11-diol is a weak dopamine antagonist for the treatment of neurological disorders. (6aS)-6-methyl-5,6,6a,7-tetrahydro-4H-dibenzo[de,g]quinoline-10,11-diol, also known as S-(+)-10,11-dihydroxyaporphine, is depicted by the following chemical structure:

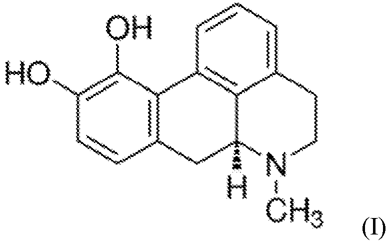

Free base of (6aS)-6-methyl-5,6,6a,7-tetrahydro-4H-dibenzo[de,g]quinoline-10,11-diol exhibited a low solubility in the aqueous media at various pH. The hydrochloride salt of (6aS)-6-methyl-5,6,6a,7-tetrahydro-4H-dibenzo[de,g]quinoline-10,11-diol also showed low solubility in the aqueous buffer solutions. There is clearly a need to develop liquid dosage formulations with higher solubility. Those novel formulations can enable parenteral delivery and provide a better safety profile. They can also be used in oral administration to achieve higher oral bioavailability or an improved clinical profile.

### Description

It must also be noted that as used herein and in the appended claims, the singular forms "a," "an," and "the" include plural reference unless the context clearly dictates otherwise. Thus, for example, reference to an "excipient" is a reference to one or more cells and equivalents thereof known to those skilled in the art, and so forth.

As used herein, the term "about" means plus or minus 5% of the numerical value of the number with which it is being used. Therefore, about 50% means in the range of 45%-55%.

"Administering" when used in conjunction with a therapeutic means to administer a therapeutic directly to a subject, whereby the agent positively impacts the target. "Administering" a composition may be accomplished by, for example, injection, oral administration, topical administration, or by these methods in combination with other known techniques. Such combination techniques include heating, radiation, ultrasound and the use of delivery agents. When a compound is provided in combination with one or more other active agents, "administration" and its variants are each understood to include concurrent and sequential provision of the compound or salt and other agents.

By "pharmaceutically acceptable" it is meant the carrier, diluent, adjuvant, or excipient must be compatible with the other ingredients of the formulation and not deleterious to the recipient thereof.

"Composition" as used herein is intended to encompass a product comprising the specified ingredients in the specified amounts, as well as any product which results, directly or indirectly, from combination of the specified ingredients in the specified amounts. Such term in relation to "pharmaceutical composition" is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound o the present invention and a pharmaceutically acceptable carrier. Percentages described herein are in terms of percent weight unless otherwise specified.

As used herein, the term "agent," "active agent," "therapeutic agent," or "therapeutic" means a compound or composition utilized to treat, combat, ameliorate, prevent or improve an unwanted condition or disease of a patient. Furthermore, the term "agent," "active agent," "therapeutic agent," or "therapeutic" encompasses a combination of one or more of the compounds of the present invention. (6aS)-6-methyl-5,6,6a,7-tetrahydro-4H-dibenzo[de,g]quinoline-10,11-diol or a pharmaceutically acceptable salt thereof may also be referred to as "Compound 1".

A "therapeutically effective amount" or "effective amount" of a composition is a predetermined amount calculated to achieve the desired effect, *i.e.,* to inhibit, block, or reverse the activation, migration, proliferation, alteration of cellular function, and to preserve the normal function of cells. The activity contemplated by the methods described herein includes both medical therapeutic and/or prophylactic treatment, as appropriate, and the compositions of the invention may be used to provide improvement in any of the conditions described. It is also contemplated that the compositions described herein may be administered to healthy subjects or individuals not exhibiting symptoms but who may be at risk of developing a particular disorder. The specific dose of a compound administered according to this invention to obtain therapeutic and/or prophylactic effects will, of course, be determined by the particular circumstances surrounding the case, including, for example, the compound administered, the route of administration, and the condition being treated. However, it will be understood that the chosen dosage ranges are not intended to limit the scope of the invention in any way. A therapeutically effective amount of compound of this invention is typically an amount such that when it is administered in a physiologically tolerable excipient composition, it is sufficient to achieve an effective systemic concentration or local concentration in the tissue.

The terms "treat," "treated," or "treating" as used herein refer to both therapeutic treatment and prophylactic or preventative measures, wherein the object is to prevent or slow down (lessen) an undesired physiological condition, disorder, or disease, or to obtain beneficial or desired clinical results. For the purposes of this invention, beneficial or desired results include, but are not limited to, alleviation of symptoms; diminishment of the extent of the condition, disorder, or disease; stabilization (i.e., not worsening) of the state of the condition, disorder, or disease; delay in onset or slowing of the progression of the condition, disorder, or disease; amelioration of the condition, disorder, or disease state; and remission (whether partial or total), whether detectable or undetectable, or enhancement or improvement of the condition, disorder, or disease. Treatment includes prolonging survival as compared to expected survival if not receiving treatment.

### Administration and Compositions

Pharmaceutical compositions comprising 6aS)-6-methyl-5,6,6a,7-tetrahydro-4H-dibenzo[de,g]quinoline-10,11-diol and pharmaceutically-acceptable salts thereof can be administered by means that produces contact of the active agent with the agent's site of action. They can be administered by conventional means available for use in conjunction with pharmaceuticals in a dosage range of 0.001 to 1000 mg/kg of mammal body weight per day in a single dose or in divided doses. One dosage range is 0.01 to 500 mg/kg body weight per day orally in a single dose or in divided doses. Administration can be delivered as individual therapeutic agents or in a combination of therapeutic agents. They can be administered alone, but typically are administered with a pharmaceutically acceptable excipient selected on the basis of the chosen route of administration and standard pharmaceutical practice.

Compounds can be administered by one or more ways. For example, the following routes may be utilized: oral, parenteral (including subcutaneous (SubQ or SC) injections, intravenous, intramuscular, intrasternal injection or infusion techniques), inhalation, buccal, intranasal, sublingual (SL), or rectal, in the form of a unit dosage of a pharmaceutical composition containing an effective amount of the compound and optionally in combination with one or more pharmaceutically-acceptable excipients such as stabilizers, anti-oxidants, lubricants, bulking agents, fillers, carriers, adjuvants, vehicles, diluents and other readily known excipients in standard pharmaceutical practice.

Liquid preparations suitable for oral administration (e.g. suspensions, syrups, elixirs and other similar liquids) can employ media such as water, glycols, oils, alcohols, and the like. Solid preparations suitable for oral administration (e.g. powders, pills, capsules and tablets) can employ solid excipients such as starches, sugars, kaolin, lubricants, binders, disintegrating agents, antioxidants and the like.

Parenteral compositions typically employ sterile water as a carrier and optionally other ingredients, such as solubility aids. Injectable solutions can be prepared, for example, using a carrier comprising a saline solution, a glucose solution or a solution containing a mixture of saline and glucose. Further guidance for methods suitable for use in preparing pharmaceutical compositions is provided in Remington: The Science and Practice of Pharmacy, 21st edition (Lippincott Williams & Wilkins, 2006).

Therapeutic compounds can be administered orally in a dosage range of about 0.001 to 1000 mg/kg of mammal body weight per day in a single dose or in divided doses. One dosage range is about 0.01 to 500 mg/kg body weight per day orally in a single dose or in divided doses. For oral administration, the compositions can be provided in a form containing about 1.0 to 500mg of the active ingredient, particularly about 1, 5, 10, 15, 20, 25, 50, 75, 100, 150, 200, 250, 300, 400, 500, and 750 mg of the active ingredient for the symptomatic adjustment of the dosage to the patient to be treated. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy. In view of the factors affecting the specific dose level and frequency it is contemplated that the dose frequency can range from multiple doses daily to monthly dosages. The preferred dose frequency ranges from twice a day to every two weeks. A more preferred dose frequency ranges from twice a day to weekly. A most preferred dose frequency ranges from twice a day to twice a week.

In the methods of various embodiments, pharmaceutical compositions including the active agent can be administered to a subject in an "effective amount." An effective amount may be any amount that provides a beneficial effect to the patient, and in particular embodiments.

Pharmaceutical formulations containing the compounds of the invention and a suitable carrier can be in various forms including, but not limited to, solids, solutions, powders, fluid emulsions, fluid suspensions, semi-solids, and dry powders including an effective amount of an the active agent of the invention. It is also known in the art that the active ingredients can be contained in such formulations with pharmaceutically acceptable diluents, fillers, disintegrants, binders, lubricants, surfactants, hydrophobic vehicles, water soluble vehicles, emulsifiers, buffers, humectants, moisturizers, solubilizers, antioxidants, preservatives, cosolvents, cyclodextrins, and the like. The means and methods for administration are known in the art and an artisan can refer to various pharmacologic references for guidance. For example, Modern Pharmaceutics, Banker & Rhodes, Marcel Dekker, Inc. (1979); and Goodman & Gilman's, The Pharmaceutical Basis of Therapeutics, 6th Edition, MacMillan Publishing Co., New York (1980).

Further embodiments which may be useful for oral administration of the active agent include liquid dosage forms. In such embodiments, a liquid dosage may include a pharmaceutically acceptable emulsion, solution, suspension, syrup, and elixir containing inert diluents commonly used in the art, such as water. Such compositions may also comprise adjuvants, such as wetting agents, emulsifying and suspending agents, and sweetening, flavoring, and perfuming agents. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

The composition of the claims include HPβCD and/or SBEβCD. In some embodiments HPβCD is included in the range of about 0.1% to about 80%, more preferably in the range of 5% to about 60%, and even more preferably in the range of about 10% to about 40%. In other embodiments SBEβCD is included in the range of about 0.1% to about 80%, more preferably in the range of 5% to about 60%, and even more preferably in the range of about 10% to about 40%.

Further, embodiments of the present invention may also include antioxidants and preservatives. Some preferred antioxidants and preservatives include: butylated hydroxyanisole, butylated hydroxytoluene, dehydroacetic acid, ethylenediamine, potassium benzoate, potassium metabisulfite, potassium sorbate, sodium bisulfite, sodium metabisulfite, and sorbic acid. In one embodiment the antioxidant or preservative is sodium metabisulfite. In one embodiment, sodium metabisulfite is included in the range of about 0.001% to about 5%, and more preferably in the range of about 0.01% to about 1%, and even more preferably in the range of about 0.1% to about 0.5%.

Embodiments of the present invention may also include cosolvents and/or buffers. Some preferred cosolvents and/or buffers include: glycerin, ethanol; about citrate buffer, pH 3.0; citrate buffer, pH 4.0; citrate buffer, pH 5.0; citrate buffer, pH 6.0; 20% HPβCD in water; 20% HPβCD in citrate buffer, pH 3.0; 20% SBEβCD in water; 20% SBEβCD in citrate buffer, pH 3.0; 20% SBEβCD in citrate buffer, pH 5.0; 10% solutol HS15 in water; 10% cremophor EL in water; 10%Vitamin ETPGS in water; 1% Tween 80 in water; 20% HPβCD in citrate buffer, pH 5.5; 20% HPβCD in citrate buffer, pH 5.0; 20% HPβCD in citrate buffer, pH 4.5; 30% HPβCD in citrate buffer, pH 5.5; 30% HPβCD in citrate buffer, pH 5.0; 30% HPβCD in citrate buffer, pH 4.5; benzyl alcohol; acetate buffer, pH 3.0; acetate buffer, pH 4.0; acetate buffer, pH 5.0; acetate buffer, pH 5.5; and acetate buffer, pH 6.0. In some embodiments the benzyl alcohol is included in the range of about 0.001% to about 20%, and more preferably in the range of about 0.01% to about 5%, and even more preferably in the range of about 0.1% to about 1%.

Polyethylene glycol: As used herein, the term "polyethylene glycol" or "PEG" refers to a polymer containing ethylene glycol monomer units of formula -O-CH₂-CH₂-. Suitable polyethylene glycols may have a free hydroxyl group at each end of the polymer molecule, or may have one or more hydroxyl groups etherified with a lower alkyl, e.g., a methyl group. Also suitable are derivatives of polyethylene glycols having esterifiable carboxy groups. Polyethylene glycols useful in the present invention can be polymers of any chain length or molecular weight, and can include branching. In some embodiments, the average molecular weight of the polyethylene glycol is from about 200 to about 9000. In some embodiments, the average molecular weight of the polyethylene glycol is from about 200 to about 5000. In some embodiments, the average molecular weight of the polyethylene glycol is from about 200 to about 900. In some embodiments, the average molecular weight of the polyethylene glycol is about 400. Suitable polyethylene glycols include, but are not limited to polyethylene glycol-200, polyethylene glycol-300, polyethylene glycol-400, polyethylene glycol-600, and polyethylene glycol-900. The number following the dash in the name refers to the average molecular weight of the polymer. In some embodiments, the polyethylene glycol is polyethylene glycol-400. Suitable polyethylene glycols include, but are not limited to the Carbowax^{™} and Carbowax^{™} Sentry series (available from Dow), the Lipoxol^{™} series (available from Brenntag), the Lutrol^{™} series (available from BASF), and the Pluriol^{™} series (available from BASF).

In some embodiments, the diluent component comprises one or more of mannitol, lactose, sucrose, maltodextrin, sorbitol, xylitol, powdered cellulose, microcrystalline cellulose, carboxymethylcellulose, carboxyethylcellulose, methylcellulose, ethylcellulose, hydroxyethylcellulose, methylhydroxyethylcellulose, starch, sodium starch glycolate, pregelatinized starch, a calcium phosphate, a metal carbonate, a metal oxide, or a metal aluminosilicate.

Pharmaceutical formulations included within the present invention generally contain about 1% to about 99% by weight of Compound 1 and 99% to 1% by weight of one or more suitable pharmaceutical excipient.

In some embodiments, a pharmaceutical composition of the present invention is delivered to a subject via a parenteral route, an enteral route, or a topical route. Examples of parental routes the present invention include, without limitation, any one or more of the following: intraabdominal, intra-amniotic, intra-arterial, intra-articular, intrabiliary, intrabronchial, intrabursal, intracardiac, intracartilaginous, intracaudal, intracavernous, intracavitary, intracerebral, intracisternal, intracorneal, intracoronal, intracoronary, intracorporus, intracranial, intradermal, intradiscal, intraductal, intraduodenal, intradural, intraepidermal, intraesophageal, intragastric, intragingival, intraileal, intralesional, intraluminal, intralymphatic, intramedullary, intrameningeal, intramuscular, intraocular, intraovarian, intrapericardial, intraperitoneal, intrapleural, intraprostatic, intrapulmonary, intraocular, intrasinal, intraspinal, intrasynovial, intratendinous, intratesticular, intrathecal, intrathoracic, intratubular, intratumoral, intratympanic, intrauterine, intravascular, intravenous (bolus or drip), intraventricular, intravesical, and/or subcutaneous.

Enteral routes of administration of the present invention include administration to the gastrointestinal tract via the mouth (oral), stomach (gastric), and rectum (rectal). Gastric administration typically involves the use of a tube through the nasal passage (NG tube) or a tube in the esophagus leading directly to the stomach (PEG tube). Rectal administration typically involves rectal suppositories. Oral administration includes sublingual and buccal administration.

Topical administration includes administration to a body surface, such as skin or mucous membranes, including intranasal and pulmonary administration. Transdermal forms include cream, foam, gel, lotion or ointment. Intranasal and pulmonary forms include liquids and powders, e.g., liquid spray.

Preparation of some preferred vehicles includes:
10% solutol HS15 in water(v/v): Place 1.0 mL of preheating solutol HS15 in a 10 mL volumetric flask and add water to volume.
10% cremophor EL in water(v/v): Place 1.0 mL of cremophor EL in a 10 mL volumetric flask and add water to volume.
10%Vitamin ETPGS in water(v/v): Place 1.0 mL of preheating Vitamin ETPGS in a 10 mL volumetric flask and add water to volume.
1%Tween 80 in water(v/v): Place 0.1mL of Tween 80 in a 10 mL volumetric flask and add water to volume.
20%HPβCD in 100mM citrate buffer, pH3.0: Place 0.5 g of HP(3CD in a 2.5 mL volumetric flask and add 100mM pH 3.0 citrate buffer to volume.
20%HPβCD in 100mM citrate buffer, pH5.0:Place 0.5 g of HPβCD in a 2.5 mL volumetric flask and add 100mM pH 5.0 citrate buffer to volume.
20%SBEβCD in water: Place 0.5 g of SBE-β-CD in a 2.5 mL volumetric flask and add water to volume.
20%SBEβCD in 100mM citrate buffer, pH3.0: Place 0.5 g of SBEβCD in a 2.5 mL volumetric flask and add 100mM pH 3.0 citrate buffer to volume.
20%SBEβCD in 100mM citrate buffer, pH5.0: Place 0.5 g of SBEβCD in a 2.5 mL volumetric flask and add 100mM pH 5.0 citrate buffer to volume.
0.15%sodium metabisulfite+0.5%benzyl alcohol in water: Place 300 mg of sodium metabisulfite and 1020 mg of benzyl alcohol in a 200 mL volumetric flask and add water to volume.
0.15%sodium metabisulfite+0.5%benzyl alcohol in 50mM citrate buffer, pH 3.0: Place 75 mg of sodium metabisulfite and 250 mg of benzyl alcohol in a 50 mL volumetric flask and add 50mM pH 3.0 citrate buffer to volume.
20%HPβCD in (0.15%sodium metabisulfite+0.5%benzyl alcohol) water: Place 5000 mg of HPβCD in a 25 mL volumetric flask and add 0.15%sodium metabisulfite+0.5%benzyl alcohol in water to volume.
20%HPβCD in (0.15%sodium metabisulfite+0.5%benzyl alcohol) 50 mM citrate buffer, pH 3.0: Place2000 mg of HPβCD in a 10 mL volumetric flask and add 0.15%sodium metabisulfite+0.5%benzyl alcohol in 50mM citrate buffer, pH 3.0 to volume.
20%SBEβCD in (0.15%sodium metabisulfite+0.5%benzyl alcohol) water:Place 5000 mg of SBEβCD in a 25 mL volumetric flask and add 0.15%sodium metabisulfite+0.5%benzyl alcohol in water to volume.
20%SBEβCD in (0.15%sodium metabisulfite+0.5%benzyl alcohol) 50 mM citrate buffer, pH 3.0: Place 2000 mg of SBEβCD in a 10 mL volumetric flask and add 0.15%sodium metabisulfite+0.5%benzyl alcohol in 50mM citrate buffer, pH 3.0 to volume.
40%HPβCD in (0.2%sodium metabisulfite+0.5%benzyl alcohol) water:Place 50 mg of sodium metabisulfite, 125 mg of benzyl alcohol and 10000 mg of HPβCD in a 25 mL volumetric flask, add 15 mL of water and sonicate for 5 mins to dissolve, and then dilute to volume with water.
40%HPβCD in (0.2%sodium metabisulfite+0.5%benzyl alcohol) 100 mM citrate buffer, pH 3.0: Place 50 mg g of sodium metabisulfite, 125 mg of benzyl alcohol and 10000 mg of HPβCD in a 25 mL volumetric flask, add 15 mL of 100 mM pH 3.0 citrate buffer and sonicate for 5 mins to dissolve, and then dilute to volume with pH 3.0 100 mM citrate buffer.
40%HPβCD in (0.2%sodium metabisulfite+0.5%benzyl alcohol) 100 mM citrate buffer, pH 4.0:Place 50 mg g of sodium metabisulfite, 125 mg of benzyl alcohol and 10000 mg of HPβCD in a 25 mL volumetric flask, add 15 mL of 100 mM pH 4.0 citrate buffer and sonicate for 5 mins to dissolve, and then dilute to volume with 100 mM pH 4.0 citrate buffer.
40%HPβCD in (0.2%sodium metabisulfite+0.5%benzyl alcohol) 200 mM citrate buffer, pH 3.0: Place 50 mg g of sodium metabisulfite, 125 mg of benzyl alcohol and 10000 mg of HPβCD in a 25 mL volumetric flask, add 15 mL of 200 mM pH 3.0 citrate buffer and sonicate for 5 mins to dissolve, and then dilute to volume with 200 mM pH 3.0 citrate buffer.
40%HPβCD in (0.2%sodium metabisulfite+0.5%benzyl alcohol) 50 mM citrate buffer, pH 3.0:Place 50 mg g of sodium metabisulfite, 125 mg of benzyl alcohol and 10000 mg of HPβCD in a 25 mL volumetric flask, add 15 mL of 50 mM pH 3.0 citrate buffer and sonicate for 5 mins to dissolve, and then dilute to volume with 50 mM pH 3.0 citrate buffer.
20%HPβCD in (0.2%sodium metabisulfite+0.5%benzyl alcohol) 50 mM citrate buffer, pH 3.0: Place 5 mL of the 40%HPβCD in (0.2%sodium metabisulfite+0.5%benzyl alcohol) 200 mM pH 3.0 citrate buffer in a 10-mL volumetric flask, add 5 mL of the 0.15%sodium metabisulfite+0.5%benzyl alcohol in water and mix well.
15%HPβCD in (0.15%sodium metabisulfite+0.5%benzyl alcohol) 100 mM citrate buffer, pH 3.0: Place 37.5 mg g of sodium metabisulfite, 125 mg of benzyl alcohol and 3750 mg of HPβCDin a 25 mL volumetric flask, add 15 mL of 100 mM pH 3.0 citrate buffer and sonicate for 5 mins to dissolve, and then dilute to volume with 100 mM pH 3.0 citrate buffer.
15%HPβCD in (0.15%sodium metabisulfite+0.5%benzyl alcohol) 100 mM citrate buffer, pH 4.0: Place 37.5 mg g of sodium metabisulfite, 125 mg of benzyl alcohol and 3750 mg of HPβCDin a 25 mL volumetric flask, add 15 mL of 100 mM pH 4.0 citrate buffer and sonicate for 5 mins to dissolve, and then dilute to volume with 100 mM pH 4.0 citrate buffer
10%HPβCD in (0.15%sodium metabisulfite+0.5%benzyl alcohol) 100 mM citrate buffer, pH 3.0: Place 37.5 mg g of sodium metabisulfite, 125 mg of benzyl alcohol and 2500 mg of HPβCDin a 25 mL volumetric flask, add 15 mL of 100 mM pH 3.0 citrate buffer and sonicate for 5 mins to dissolve, and then dilute to volume with pH 3.0 100 mM citrate buffer.
10%HPβCD in (0.15%sodium metabisulfite+0.5%benzyl alcohol) 100 mM citrate buffer, pH 4.0: Place 37.5 mg g of sodium metabisulfite, 125 mg of benzyl alcohol and 2500 mg of HPβCDin a 25 mL volumetric flask, add 15 mL of pH4.0 100 mM citrate buffer and sonicate for 5 mins to dissolve, and then dilute to volume with 100 mM pH 4.0 citrate buffer.
100mM acetate buffer, pH 3.6:Place 36.9mg g of acetate sodium, 585.3 mg of acetic acid in a 100 mL volumetric flask, add 90 mL ofwater for 5 mins to dissolve, adjust the pH to 3.6 with 1N HCl or 1N NaOH, and then dilute to volume with water.
100mM acetate buffer, pH 4.5:Place 369.6 mg g of acetate sodium, 190.67 mg of acetic acid in a 100 mL volumetric flask, add 90 mL ofwater for 5 mins to dissolve, adjust the pH to 4.5 with 1N HCl or 1N NaOH, and then dilute to volume with water.
100mM acetate buffer, pH 5.0:Place553.4 mg g of acetate sodium, 328.7 mg of acetic acid in a 100 mL volumetric flask, add 90 mL ofwater for 5 mins to dissolve, adjust the pH to 5.0 with 1N HCl or 1N NaOH, and then dilute to volume with water.
100mM acetate buffer, pH 5.5:Place734.9 mg g of acetate sodium, 65.3 mg of acetic acid in a 100 mL volumetric flask, add 90 mL ofwater for 5 mins to dissolve, adjust the pH to 5.5 with 1N HCl or 1N NaOH, and then dilute to volume with water.
20%HPβCD in (0.15%sodium metabisulfite+0.5%benzyl alcohol) 100 mM acetate, pH 3.6: Place 75 mg g of sodium metabisulfite,250 mg of benzyl alcohol and 10000 mg of HPβCD in a 25 mL volumetric flask, add 100mM acetate buffer, pH 3.6 and sonicate for 5 mins to dissolve, and then dilute to volume with 100mM acetate buffer, pH 3.6.
20%HPβCD in (0.15%sodium metabisulfite+0.5%benzyl alcohol) 100 mM acetate, pH 4.5: Place 75 mg g of sodium metabisulfite,250 mg of benzyl alcohol and 10000 mg of HPβCD in a 25 mL volumetric flask, add 100mM pH4.5 acetate buffer and sonicate for 5 mins to dissolve, and then dilute to volume with 100mM pH4.5 acetate buffer.
20%HPβCD in (0.15%sodium metabisulfite+0.5%benzyl alcohol) 100 mM acetate, pH 5.0: Place 75 mg g of sodium metabisulfite,250 mg of benzyl alcohol and 10000 mg of HPβCD in a 25 mL volumetric flask, add 100mM pH 5.0 acetate buffer and sonicate for 5 mins to dissolve, and then dilute to volume with 100mM pH 5.0 acetate buffer.
20%HPβCD in (0.15%sodium metabisulfite+0.5%benzyl alcohol) 100 mM acetate, pH 5.5: Place 75 mg g of sodium metabisulfite,250 mg of benzyl alcohol and 10000 mg of HPβCD in a 25 mL volumetric flask, add 100mM pH 5.5 acetate buffer and sonicate for 5 mins to dissolve, and then dilute to volume with 100mM pH 5.5 acetate buffer.
25mM citrate buffer, pH 5.0: Place 211mg g of sodium citrate dihydrate, 102 mg of citric acid in a 50mL volumetric flask, add 45 mL of water for 5 mins to dissolve, adjust the pH to 5.0 with 1N HCl or 1N NaOH, and then dilute to volume with water.
25mM acetate buffer, pH 5.5: Place 95mg g of acetate sodium, 7.3 mg of acetic acid in a 50 mL volumetric flask, add 45 mL of water for 5 mins to dissolve, adjust the pH to 5.5 with 1N HCl or 1N NaOH, and then dilute to volume with water.
20%HPβCD in (0.15%sodium metabisulfite+0.5%benzyl alcohol) 25mM citrate buffer, pH 5.0: Place 75 mg of sodium metabisulfite,250 mg of benzyl alcohol and 10000 mg of HPβCD in a 25 mL volumetric flask, add 25mM pH 5.0 acetate buffer and sonicate for 5 mins to dissolve, and then dilute to volume with 25mM pH 5.5 acetate buffer.
20%HPβCD in (0.15%sodium metabisulfite+0.5%benzyl alcohol) 25 mM acetate, pH 5.5: Place 75 mg of sodium metabisulfite,250 mg of benzyl alcohol and 10000 mg of HPβCD in a 25 mL volumetric flask, add 25mM pH 5.5 acetate buffer and sonicate for 5 mins to dissolve, and then dilute to volume with 25mM acetate buffer, pH5.5.
15mM acetate buffer, pH 5.5: Place 55mg g of acetate sodium, 5.3 mg of acetic acid in a 50 mL volumetric flask, add 45 mL of water for 5 mins to dissolve, adjust the pH to 5.5 with 1N HCl or 1N NaOH, and then dilute to volume with water.
15mM citrate buffer, pH 5.5: Place 158mg g of sodium citrate dihydrate, 43 mg of citric acid in a 50mL volumetric flask, add 45 mL of water for 5 mins to dissolve, adjust the pH to 5.5 with 1N HCl or 1N NaOH, and then dilute to volume with water.
20%HPβCD in (0.15%sodium metabisulfite+0.5%benzyl alcohol) 15 mM acetate, pH 5.5: Place 75 mg g of sodium metabisulfite,250 mg of benzyl alcohol and 10000 mg of HPβCD in a 25 mL volumetric flask, add 15 mM pH 5.5 acetate buffer and sonicate for 5 mins to dissolve, and then dilute to volume with 15mM pH 5.5 acetate buffer.
20%HPβCD in (0.15%sodium metabisulfite+0.5%benzyl alcohol) 15mM citrate buffer, pH 5.5: Place 75 mg of sodium metabisulfite,250 mg of benzyl alcohol and 10000 mg of HPβCD in a 25 mL volumetric flask, add 15mM pH 5.0 citrate buffer and sonicate for 5 mins to dissolve, and then dilute to volume with 15mM pH 5.5 citrate buffer.
10mM citrate buffer, pH 5.5: Place 0.2053 g of sodium citrate dihydrate, 0.058 g of citric acid in a 100mL volumetric flask, add 80 mL of water for 5 mins to dissolve, adjust the pH to 5.5 with 1N HCl or 1N NaOH, and then dilute to volume with water.
10mM citrate buffer, pH 5.0: Place 0.1696 g of sodium citrate dihydrate, 0.0813 g of citric acid in a 100mL volumetric flask, add 80 mL of water for 5 mins to dissolve, adjust the pH to 5.0 with 1N HCl or 1N NaOH, and then dilute to volume with water.
10mM citrate buffer, pH 4.5: Place 0.134 g of sodium citrate dihydrate, 0.1045 g of citric acid in a 100mL volumetric flask, add 80 mL of water for 5 mins to dissolve, adjust the pH to 4.5 with 1N HCl or 1N NaOH, and then dilute to volume with water.
10mM citrate buffer, pH 6.0: Place 0.241g sodium citrate dihydrate, 0.035 g of citric acid in a 100mL volumetric flask, add 80 mL of water for 5 mins to dissolve, adjust the pH to 6.0 with 1N HCl or 1N NaOH, and then dilute to volume with water.
25%HPβCD in (0.15%sodium metabisulfite+0.5%benzyl alcohol) 10mM citrate buffer, pH 5.5: Place 15 mg of sodium metabisulfite,50 mg of benzyl alcohol and 2.5 g of HPβCD in a 10 mL volumetric flask, add 8mL of 10mM pH 5.5 citrate buffer and sonicate for 5 mins to dissolve, and then dilute to volume with buffer.
25%HPβCD in (0.15%sodium metabisulfite+0.5%benzyl alcohol) 10mM citrate buffer, pH 5.0: Place 15 mg of sodium metabisulfite,50 mg of benzyl alcohol and 2.5 g of HPβCD in a 10 mL volumetric flask, add 8mL of 10mM pH 5.0 citrate buffer and sonicate for 5 mins to dissolve, and then dilute to volume with buffer.
25%HPβCD in (0.15%sodium metabisulfite+0.5%benzyl alcohol) 10mM citrate buffer, pH 4.5: Place 15 mg of sodium metabisulfite,50 mg of benzyl alcohol and 2.5 g of HPβCD in a 10 mL volumetric flask, add 8mL of 10mM pH 4.5 citrate buffer and sonicate for 5 mins to dissolve, and then dilute to volume with buffer.
30%HPβCD in (0.15%sodium metabisulfite+0.5%benzyl alcohol) 10mM citrate buffer, pH 5.5: Place 15 mg of sodium metabisulfite,50 mg of benzyl alcohol and 3.0 g of HPβCD in a 10 mL volumetric flask, add 8mL of 10mM pH 5.5 citrate buffer and sonicate for 5 mins to dissolve, and then dilute to volume with buffer.
30%HPβCD in (0.15%sodium metabisulfite+0.5%benzyl alcohol) 10mM citrate buffer, pH 5.0: Place 15 mg of sodium metabisulfite,50 mg of benzyl alcohol and 3.0 g of HPβCD in a 10 mL volumetric flask, add 8mL of 10mM pH 5.0 citrate buffer and sonicate for 5 mins to dissolve, and then dilute to volume with buffer.
30%HPβCD in (0.15%sodium metabisulfite+0.5%benzyl alcohol) 10mM citrate buffer, pH 4.5: Place 15 mg of sodium metabisulfite,50 mg of benzyl alcohol and 3.0 g of HPβCD in a 10 mL volumetric flask, add 8mL of 10mM pH 4.5 citrate buffer and sonicate for 5 mins to dissolve, and then dilute to volume with buffer.
100mM citrate buffer, pH 5.5:Place 2.053 g of sodium citrate dihydrate, 0.58 g of citric acid in a 100mL volumetric flask, add 80 mL of water for 5 mins to dissolve, adjust the pH to 5.5 with 1N HCl or 1N NaOH, and then dilute to volume with water.
100mM citrate buffer, pH 5.0: Place 1.696 g of sodium citrate dihydrate, 0.813 g of citric acid in a 100mL volumetric flask, add 80 mL of water for 5 mins to dissolve, adjust the pH to 5.0 with 1N HCl or 1N NaOH, and then dilute to volume with water.
100mM citrate buffer, pH 4.5: Place 1.34 g of sodium citrate dihydrate, 1.045 g of citric acid in a 100mL volumetric flask, add 80 mL of water for 5 mins to dissolve, adjust the pH to 4.5 with 1N HCl or 1N NaOH, and then dilute to volume with water.

### Examples

### Example 1: Preparation of (6aS)-6-methyl-5,6,6a,7-tetrahydro-4H-dibenzo[de,g]quinoline-10,11-diol Hydrochloride

The following examples contain detailed methods of preparing (6aS)-6-methyl-5,6,6a,7-tetrahydro-4H-dibenzo[de,g]quinoline-10,11-diol. The general synthetic scheme is presented for illustrative purposes only and is not intended as a restriction on the scope of the invention. All parts are by weight and temperatures are in Degrees Celsius unless otherwise indicated.

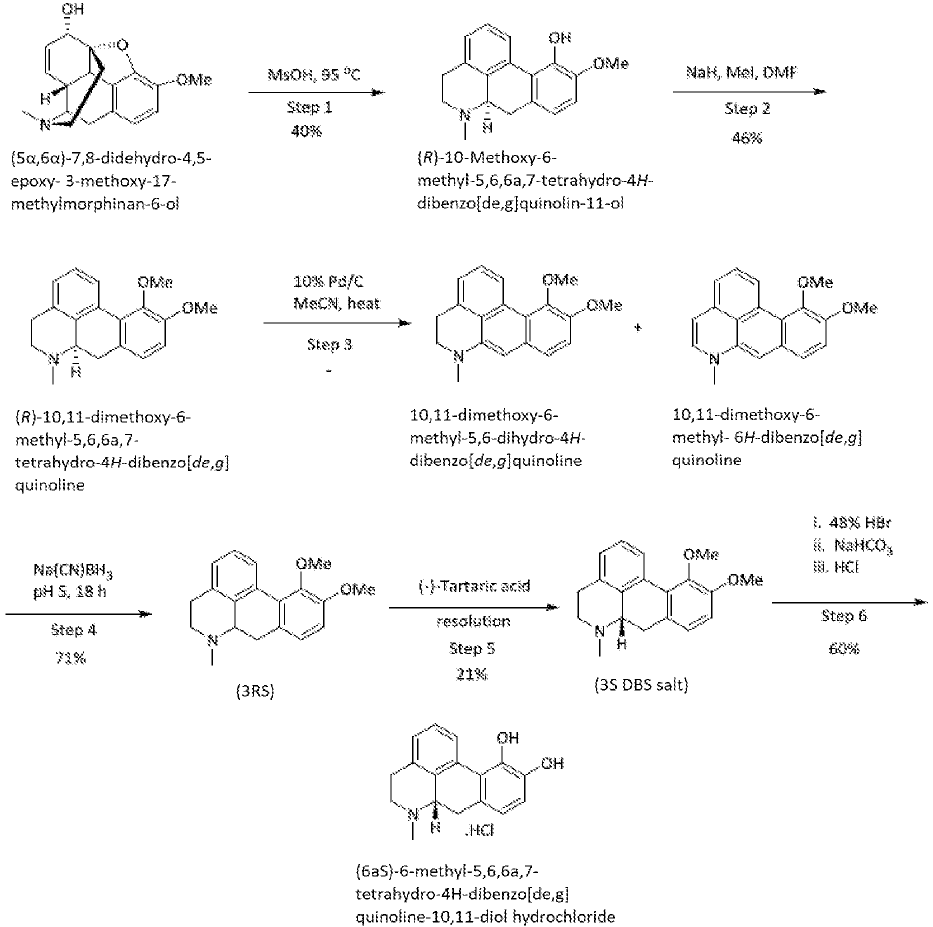

### Preparation of (R)-10-Methoxy-6-methyl-5,6,6a,7-tetrahydro-4H-dibenzo[de,g]quinolin-11-ol

A mixture of(5α,6α)-7,8-didehydro-4,5-epoxy- 3-methoxy-17-methylmorphinan-6-ol (10.0 g, 33.4 mmol) and methanesulfonic acid (40 ml) was heated to 100 °C. After 30 minutes, the temperature was lowered to 65 °C and after a further 30 minutes, the reaction was left to cool to room temperature. The mixture was diluted with water (150 ml) and then basified with ammonium hydroxide (with cooling in an ice bath to keep the temperature < 50 °C). The resultant suspension was cooled to room temperature, collected by filtration and washed with water. The solid was dissolved in dichloromethane (50 ml) and separated from residual water. The resultant solution was passed through a pad of silica eluting with 5% methanol/ dichloromethane. Concentration of appropriate fractions gave the desired product as a white foam (3.8 g, 40%).

### Preparation of (R)-10,11-dimethoxy-6-methyl-5,6,6a,7-tetrahydro-4H-dibenzo[de,g]quinoline

To a solution of (R)-10-Methoxy-6-methyl-5,6,6a,7-tetrahydro-4H-dibenzo[de,g]quinolin-11-ol (5.0 g, 17.8 mmol) in dimethylformamide (25 ml) was added 60 % sodium hydride (0.75 g, 18.8 mmol). After stirring for 20 minutes, methyl iodide (2.66 g, 18.8 mmol) was added dropwise over 10 minutes. The reaction mixture was stirred for 20 minutes and then poured onto water (70 ml) and extracted with methyl t-butyl ether (150 ml). The organic layer was separated, washed with 2M sodium hydroxide solution (3 x 50 ml) then brine (50 ml) and concentrated to a pink oil which was purified on a plug of silica eluted 5% methanol/ dichloromethane. Concentration of appropriate fractions gave the desired product (2.43 g, 46%).

### Preparation of 10,11-dimethoxy-6-methyl-5,6-dihydro-4H-dibenzo [de,g]quinoline and 10,11-dimethoxy-6-methyl-6H-dibenzo[de,g]quinoline

A solution of (R)-10,11-dimethoxy-6-methyl-5,6,6a,7-tetrahydro-4H-dibenzo[de,g]quinoline (18.47 g, 65.9 mmol) in acetonitrile (550 ml) was treated with a slurry of 10% Pd/C in acetonitrile (450 ml) and the mixture heated at reflux overnight and then allowed to cool. The catalyst was collected by filtration, washed with acetonitrile and the combined filtrate concentrated to a dark oil (10.6 g) which was used without further purification.

### Preparation of (3RS)

A mixture of 10,11-dimethoxy-6-methyl-5,6-dihydro-4H-dibenzo[de,g]quinoline and 10, II-dimethoxy-6-methyl-6H-dibenzo[de,g]quinoline (10.6 g, 36.1 mmol) was dissolved in absolute ethanol (500 ml) and treated with sodium cyanoborohoydride (11 g, 175 mmol). Part of a solution of acetyl chloride (8.8 g, 0.113 mol) in absolute ethanol (100 ml) was added until the mixture became cloudy and the mixture stirred at room temperature. After stirring overnight, the reaction was found to be around 80 % complete by LC-MS; stirring was continued and further aliquots of the acetyl chloride solution added at intervals. Once the reaction was found to contain < 2% starting material by LCMS, the mixture was concentrated and the residue diluted with water (300 ml) and basified with potassium carbonate. After extraction with ethyl acetate (2 x 300 ml), the organic layer was washed with brine, concentrated and then purified by chromatography (silica gel, 3 → 10% methanol: dichloromethane) (7.53 g, 71%).

### Preparation of (S)-10,11-dimethoxy-6-methyl-5,6,6a,7-tetrahydro-4H-dibenzo[de,g]quinoline (2R,3R)-2,3-bis(benzoyloxy)succinate (3S DBS salt)

To a solution of (-)-Dibenzoyl-L-tartaric acid (8.73 g, 24.4 mmol) in ethyl acetate (80 ml) was added a solution of (3RS) (7.20 g, 24.4 mmol) in ethyl acetate (30 ml) over 15 minutes. The resultant precipitate was heated to reflux and isopropanol (56 ml) added to give a dark solution. After heating for 15 minutes, the solution was allowed to slowly cool to room temperature for two hours. It was then further cooled in an ice bath for an hour and the resultant precipitate collected by filtration (2.6 g). A second crop was also collected (1.4 g). The two were combined and heated to reflux in ethyl acetate (35 ml); isopropanol (20 ml) was then added slowly until a solution was obtained. This was allowed to slowly cool and the product collected by filtration in 3 crops, all of which had > 95 % ee by chiral NMR (3.28 g, 21 %).

### Preparation of (6aS)-6-methyl-5,6,6a,7-tetrahydro-4H-dibenzo[de,g]quinoline-10,11-diol hydrochloride

A slurry of (3S DBS salt) (4.4 g, 6.7 mmol) was treated with potassium carbonate until pH 8. The mixture was extracted with ethyl acetate (2 x 40 ml) and the combined extracts dried and concentrated to an orange / brown oil (1.90 g). This was dissolved in acetic acid (30 ml), treated with 48% hydrobromic acid (30 ml) and the mixture heated under reflux for 5 hours under nitrogen. After cooling to room temperature overnight, the mixture was concentrated to ca 10 ml and then carefully basified by the slow addition of saturated sodium bicarbonate solution. The product was extracted with ethyl acetate (2 x 60 ml), washed with brine, dried over sodium sulphate and concentrated. The resultant blue foam was dissolved in absolute ethanol (40 ml) and treated with concentrated hydrochloric acid (1.1 ml) and activated carbon (600 mg). After heating under reflux for 40 minutes, the mixture was hot filtered and the filter bed washed with boiling absolute ethanol (80 ml). The combined filtrate was concentrated and the white solid thus obtained triturated with cold acetone (15 ml) to give the desired product. (1.08 g, 60%).

### Pharmaceutical Compositions

### Example 2: Compositions

These detailed examples are presented for illustrative purposes only and are not intended as a restriction on the scope of the invention.

About 40 mg of (6aS)-6-methyl-5,6,6a,7-tetrahydro-4H-dibenzo[de,g]quinoline-10,11-diol was weighed and transferred to a 2.0 mL vial with the target concentration of 40 mg/ml. Then 1.0 mL of different vehicles were added into the vial to dissolve the compound, the compound was sonicated for 5 min at 25°C. The pharmaceutical compositions that demonstrated an initial 40 mg/mL solubility of 6aS)-6-methyl-5,6,6a,7-tetrahydro-4H-dibenzo[de,g]quinoline-10,11-diol are detailed in the table below:

| **Formulation** | **Component** | **Drugloading (mg/mL)*** | **Final pH** | **Osmolality (mOsm/kg)** | **Appearance** |
|---|---|---|---|---|---|
| F1 | 20%HPβCD in (0.15%sodium metabisulfite+0. 5%benzyl alcohol in water) | 40 | 3.90 | 292 | Clear solution |
| F2 | 20%HPβCD in (0.15%sodium metabisulfite+0.5%benzyl alcohol in 50 mM pH3.0 citrate buffer) | 40 | 3.03 | 359 | Clear solution |
| F3 | 20%SBEβCD in (0.15%sodium metabisulfite+0.5%benzyl alcohol in water) | 40 | 2.83 | 746 | Clear solution |
| F4 | 20% SBEβCD in (0.15%sodium metabisulfite+0.5%benzyl alcohol in 50 mM pH3.0 citrate buffer) | 40 | 2.96 | 793 | Clear Solution |
| F5 | 40%HPβCD in (0.2%sodium metabisulfite+0.5%benzyl alcohol in water) | 40 | 3.79 | 741 | Clear Solution |
| F6 | 40%HPβCD in (0.2%sodium metabisulfite+0.5%benzyl alcohol in 100 mM pH3.0 citrate buffer) | 40 | 3.56 | 773 | Clear Solution |
| F7 | 40%HPβCD in (0.2%sodium metabisulfite+0.5%benzyl alcohol in 100 mM pH4.0 citrate buffer) | 40 | 4.09 | 860 | Clear Solution |
| F8 | 40%HPβCD in (0.2%sodium metabisulfite+0.5%benzyl alcohol in 200 mM pH3.0 citrate buffer) | 40 | 3.20 | 881 | Clear Solution |
| F9 | 40%HPβCD in (0.2%sodium F10metabisulfite+0.5 %benzyl alcohol in 50 mM pH3.0 citrate buffer) | 40 | 3.28 | 705 | Clear Solution |
| F10 | 25%HPβCD in (0.15%sodium metabisulfite+0.5%benzyl alcohol in water | 40 | 4.04 | 309 | Clear Solution |
| F11 | 20%HPβCD in (0.2%sodium metabisulfite+0.5%benzyl alcohol in 100 mM pH3.0 citrate buffer) | 40 | 3.03 | 381 | Clear Solution |
| F12 | 15%HPβCD in (0.15%sodium metabisulfite+0.5%benzyl alcohol) 100 mM citrate buffer, pH 3.0 | 40 | 3.01 | 362 | Clear Solution |
| F13 | 15%HPβCD in (0.15%sodium metabisulfite+0.5%benzyl alcohol) 100 mM citrate buffer, pH 4.0 | 40 | 3.99 | 424 | Clear Solution |
| F14 | 10%HPβCD in (0.15%sodium metabisulfite+0.5%benzyl alcohol) 100 mM citrate buffer, pH 3.0 | 40 | 2.98 | 335 | Clear Solution |
| F15 | 10%HPβCD in (0.15%sodium metabisulfite+0.5%benzyl alcohol) 100 mM citrate buffer, pH 4.0 | 40 | 3.93 | 337 | Clear Solution |
| F16 | 20%HPβCD in (0.15%sodium metabisulfite+0.5%benzyl alcohol) 100 mM acetate buffer, pH 3.6 | 40 | 3.35 | 399 | Clear Solution |
| F17 | 20%HPβCD in (0.15%sodium metabisulfite+0.5%benzyl alcohol) 100 mM acetate buffer, pH 4.5 | 40 | 4.52 | 447 | Clear Solution |
| F18 | 20%HPβCD in (0.15%sodium metabisulfite+0.5%benzyl alcohol) 100 mM acetate buffer, pH 5.0 | 40 | 4.90 | 463 | Clear solution |
| F19 | 20%HPβCD in (0.15%sodium metabisulfite+0. 5 %benzyl alcohol) 100 mM acetate, pH 5.5 | 40 | 5.41 | 490 | Clear solution |
| F20 | 20%HPβCD in (0.15%sodium metabisulfite+0.5%benzyl alcohol) 25mM citrate buffer, pH 5.0 | 40 | 4.89 | 367 | Clear solution |
| F21 | 20%HPβCD in (0.15%sodium metabisulfite+0.5%benzyl alcohol) 25 mM acetate buffer, pH 5.5 | 40 | 5.18 | 345 | Clear solution |
| F22 | 20%HPβCD in (0.15%sodium metabisulfite+0.5%benzyl alcohol) 15 mM acetate buffer, pH 5.5 | 40 | 5.08 | 329 | Clear solution |
| F23 | 20%HPβCD in (0.15%sodium metabisulfite+0.5%benzyl alcohol)15mM citrate buffer, pH 5.5 | 40 | 5.16 | 344 | Clear solution |
| F24 | 25%HPβCD in (0.15%sodium metabisulfite+0.5%benzyl alcohol) 10 mM citrate buffer, pH 5.5 | 40 | 5.07 | 342 | Clear solution |
| F25 | 25%HPβCD in (0.15%sodium metabisulfite+0.5%benzyl alcohol) 10 mM citrate buffer, pH 5.0 | 40 | 4.73 | 338 | Clear solution |
| F26 | 25%HPβCD in (0.15%sodium metabisulfite+0.5%benzyl alcohol) 10 mM citrate buffer, pH 4.5 | 40 | 4.30 | 333 | Clear solution |
| F27 | 30%HPβCD in (0.15%sodium metabisulfite+0.5%benzyl alcohol) 10 mM citrate buffer, pH 5.5 | 40 | 5.10 | 383 | Clear solution |
| F28 | 30%HPβCD in (0.15%sodium metabisulfite+0.5%benzyl alcohol) 10 mM citrate buffer, pH 5.0 | 40 | 4.74 | 391 | Clear solution |
| F29 | 30%HPβCD in (0.15%sodium metabisulfite+0.5%benzyl alcohol) 10 mM citrate buffer, pH 4.5 | 40 | 4.34 | 383 | Clear solution |
| F30 | 25% HPβCD in (0.3% sodium metabisulfite+0.5%benzyl alcohol) in pH6.0 10mM citrate buffer | 40 | 5.12 | 419 | Clear solution |
| F31 | 25% HPβCD in (0.3% sodium metabisulfite+0.5%benzyl alcohol) in pH5.5 10mM citrate buffer | 40 | 4.77 | 393 | Clear solution |
| F32 | 25% HPβCD in (0.3% sodium metabisulfite+0.5%benzyl alcohol) in pH5.0 10mM citrate buffer | 40 | 4.45 | 391 | Clear solution |
| F33 | 20% HPβCD in (0.3% sodium metabisulfite+0.5%benzyl alcohol) in pH6.0 10mM citrate buffer | 40 | 5.13 | 361 | Clear solution |
| F34 | 20% HPβCD in (0.3% sodium metabisulfite+0.5%benzyl alcohol) in pH5.5 10mM citrate buffer | 40 | 4.79 | 357 | Clear solution |
| F35 | 20% HPβCD in (0.3% sodium metabisulfite+0.5%benzyl alcohol) in pH5.0 10mM citrate buffer | 40 | 4.49 | 354 | Clear solution |

## Claims

1. A pharmaceutical composition comprising (6aS)-6-methyl-5,6,6a,7-tetrahydro-4H-dibenzo[de,g]quinoline-10,11-diol or salt forms thereof and at least one pharmaceutically acceptable excipient wherein the at least one pharmaceutical excipient comprises the cyclodextrin HPβCD or SBEβCD.

2. The pharmaceutical composition of claim 1, wherein HPβCD is in the range of about 0.1% to about 80% by weight, about 5% to about 60%, or about 10% to about 40%, wherein the term "about" means plus or minus 5% of the numerical value.

3. The pharmaceutical composition of claim 1, wherein SBEβCD in the range of about 0.1% to about 80% by weight, about 5% to about 60%, or about 10% to about 40%, wherein the term "about" means plus or minus 5% of the numerical value.

4. The pharmaceutical composition of claim 1, wherein the pharmaceutically acceptable excipient is an antioxidant or preservative.

5. The pharmaceutical composition of claim 4, wherein the pharmaceutically acceptable excipient is sodium metabisulfite at a concentration of about 0.001% to about 5% by weight, about 0.01% to about 1%, or about 0.1% to about 0.5%, wherein the term "about" means plus or minus 5% of the numerical value.

6. The pharmaceutical composition of claim 1, wherein the pharmaceutically acceptable excipient is a cosolvent or buffer.

7. The pharmaceutical composition of claim 6, wherein the buffer is a citrate buffer or acetate buffer.

8. The pharmaceutical composition of claim 6, wherein the cosolvent or buffer is an alcohol such as benzyl alcohol.

9. The pharmaceutical composition of claim 8, wherein benzyl alcohol is in the range of about 0.001% to about 20% by weight, about 0.01% to about 5%, or about 0.1% to about 1%, wherein the term "about" means plus or minus 5% of the numerical value.

10. A pharmaceutical composition according to claim 1 comprising (6aS)-6-methyl-5,6,6a,7-tetrahydro-4H-dibenzo[ de,g]quinoline-10, 11-diol or salt forms thereof, an antioxidant or preservative, a cyclodextrin HPβCD or SBEβCD, a cosolvent, and a buffer.

11. The pharmaceutical composition of claim 10, wherein the antioxidant or preservative is sodium metabisulfite, the cyclodextrin is HPβCD, the cosolvent is benzyl alcohol, and the buffer is a citrate buffer.

12. The pharmaceutical composition of claim 10, wherein the antioxidant or preservative is sodium metabisulfite, the cyclodextrin is SBEβCD, the cosolvent is benzyl alcohol, and the buffer is a citrate buffer.

## Patentansprüche

1. Pharmazeutische Zusammensetzung, umfassend (6aS)-6-Methyl-5,6,6a,7-tetrahydro-4H-dibenzo[de,g]chinolin-10,11-diol oder Salzformen davon und mindestens einen pharmazeutisch verträglichen Hilfsstoff, wobei der mindestens eine pharmazeutische Hilfsstoff das Cyclodextrin HPβCD oder SBEβCD umfasst.

2. Pharmazeutische Zusammensetzung nach Anspruch 1, wobei HPβCD im Bereich von etwa 0,1 bis etwa 80 Gew.-%, etwa 5 % bis etwa 60 % oder etwa 10 % bis etwa 40 % liegt, wobei der Begriff "etwa" plus oder minus 5 % des numerischen Wertes bedeutet.

3. Pharmazeutische Zusammensetzung nach Anspruch 1, wobei SBEβCD im Bereich von etwa 0,1 bis etwa 80 Gew.-%, etwa 5 % bis etwa 60 % oder etwa 10 % bis etwa 40 % liegt, wobei der Begriff "etwa" plus oder minus 5 % des numerischen Wertes bedeutet.

4. Pharmazeutische Zusammensetzung nach Anspruch 1, wobei der pharmazeutisch verträgliche Hilfsstoff ein Antioxidans oder Konservierungsmittel ist.

5. Pharmazeutische Zusammensetzung nach Anspruch 4, wobei der pharmazeutisch verträgliche Hilfsstoff Natriummetabisulfit in einer Konzentration von etwa 0,001 bis etwa 5 Gew.-%, etwa 0,01 % bis etwa 1 % oder etwa 0,1 % bis etwa 0,5 % ist, wobei der Begriff "etwa" plus oder minus 5 % des numerischen Wertes bedeutet.

6. Pharmazeutische Zusammensetzung nach Anspruch 1, wobei der pharmazeutisch verträgliche Hilfsstoff ein Colösungsmittel oder ein Puffer ist.

7. Pharmazeutische Zusammensetzung nach Anspruch 6, wobei der Puffer ein Citratpuffer oder Acetatpuffer ist.

8. Pharmazeutische Zusammensetzung nach Anspruch 6, wobei das Colösungsmittel oder der Puffer ein Alkohol wie Benzylalkohol ist.

9. Pharmazeutische Zusammensetzung nach Anspruch 8, wobei Benzylalkohol im Bereich von etwa 0,001 bis etwa 20 Gew.-%, etwa 0,01 % bis etwa 5 % oder etwa 0,1 % bis etwa 1 % liegt, wobei der Begriff "etwa" plus oder minus 5 % des numerischen Wertes bedeutet.

10. Pharmazeutische Zusammensetzung nach Anspruch 1, umfassend (6aS)-6-Methyl-5,6,6a,7-tetrahydro-4H-dibenzo[de,g]chinolin-10,11-diol oder Salzformen davon, ein Antioxidans oder Konservierungsmittel, ein Cyclodextrin HPβCD oder SBEβCD, ein Colösungsmittel und einen Puffer.

11. Pharmazeutische Zusammensetzung nach Anspruch 10, wobei das Antioxidans oder Konservierungsmittel Natriummetabisulfit ist, das Cyclodextrin HPβCD ist, das Colösungsmittel Benzylalkohol ist und der Puffer ein Citratpuffer ist.

12. Pharmazeutische Zusammensetzung nach Anspruch 10, wobei das Antioxidans oder Konservierungsmittel Natriummetabisulfit ist, das Cyclodextrin SBEβCD ist, das Colösungsmittel Benzylalkohol ist und der Puffer ein Citratpuffer ist.

## Revendications

1. Composition pharmaceutique comprenant du (6aS)-6-méthyl-5,6,6a,7-tétrahydro-4H-dibenzo[de,g]quinoléine-10,11-diol ou des formes de sel de celui-ci et au moins un excipient pharmaceutiquement acceptable, dans laquelle l'au moins un excipient pharmaceutique comprend la cyclodextrine HPβCD ou SBEβCD.

2. Composition pharmaceutique selon la revendication 1, dans laquelle la HPβCD se trouve dans la plage d'environ 0,1 % à environ 80 % en poids, d'environ 5 % à environ 60 % ou d'environ 10 % à environ 40 %, le terme « environ » signifiant plus ou moins 5 % de la valeur numérique.

3. Composition pharmaceutique selon la revendication 1, dans laquelle la SBEβCD se trouve dans la plage d'environ 0,1 % à environ 80 % en poids, d'environ 5 % à environ 60 % ou d'environ 10 % à environ 40 %, le terme « environ » signifiant plus ou moins 5 % de la valeur numérique.

4. Composition pharmaceutique selon la revendication 1, dans laquelle l'excipient pharmaceutiquement acceptable est un antioxydant ou un conservateur.

5. Composition pharmaceutique selon la revendication 4, dans laquelle l'excipient pharmaceutiquement acceptable est le métabisulfite de sodium en une concentration d'environ 0,001 % à environ 5 % en poids, d'environ 0,01 % à environ 1 % ou d'environ 0,1 % à environ 0,5 %, le terme « environ » signifiant plus ou moins 5 % de la valeur numérique.

6. Composition pharmaceutique selon la revendication 1, dans laquelle l'excipient pharmaceutiquement acceptable est un cosolvant ou un tampon.

7. Composition pharmaceutique selon la revendication 6, dans laquelle le tampon est un tampon citrate ou un tampon acétate.

8. Composition pharmaceutique selon la revendication 6, dans laquelle le cosolvant ou le tampon est un alcool tel que l'alcool benzylique.

9. Composition pharmaceutique selon la revendication 8, dans laquelle l'alcool benzylique se trouve dans la plage d'environ 0,001 % à environ 20 % en poids, d'environ 0,01 % à environ 5 % ou d'environ 0,1 % à environ 1 %, le terme « environ » signifiant plus ou moins 5 % de la valeur numérique.

10. Composition pharmaceutique selon la revendication 1 comprenant du (6aS)-6-méthyl-5,6,6a,7-tétrahydro-4H-dibenzo[de,g]quinoléine-10,11-diol ou des formes de sel de celui-ci, un antioxydant ou un conservateur, une cyclodextrine HPβCD ou SBEβCD, un cosolvant et un tampon.

11. Composition pharmaceutique selon la revendication 10, dans laquelle l'antioxydant ou le conservateur est le métabisulfite de sodium, la cyclodextrine est la HPβCD, le cosolvant est l'alcool benzylique et le tampon est un tampon citrate.

12. Composition pharmaceutique selon la revendication 10, dans laquelle l'antioxydant ou le conservateur est le métabisulfite de sodium, la cyclodextrine est la SBEβCD, le cosolvant est l'alcool benzylique et le tampon est un tampon citrate.
